# EUROPEAN PATENT APPLICATION

(11) **EP 4 159 219 A1**
(43) Date of publication of application: **05.04.2023**
(21) Application number: 21776072.7
(22) Date of filing: 23.03.2021
(51) Int. Cl.: A61K 31/7004, A23K 20/163, A23L 33/125, A61P 35/00

(54) **COMPOSITION WHICH IS FOR TREATING OR PREVENTING RENAL CELL CANCER AND CONTAINS D-ALLOSE AS ACTIVE INGREDIENT, AND METHOD FOR TREATING OR PREVENTING CANCER USING SAME**

(30) Priority: 23.03.2020 JP 2020051807
(71) Applicant: National University Corporation Kagawa University, Takamatsu-shi, Kagawa 760-8521 (JP)
(72) Inventor: TAOKA, Rikiya, Kita-gun, Kagawa 761-0793 (JP); KAKEHI, Yoshiyuki, Takamatsu-shi, Kagawa 760-8521 (JP); SUGIMOTO, Mikio, Kita-gun, Kagawa 761-0793 (JP); ZHANG, Xia, Kita-gun, Kagawa 761-0793 (JP); MATSUOKA, Yuki, Kita-gun, Kagawa 761-0793 (JP); IZUMORI, Ken, Takamatsu-shi, Kagawa 760-8521 (JP); YOSHIHARA, Akihide, Takamatsu-shi, Kagawa 760-8521 (JP)
(74) Representative: AWA Sweden AB
(86) International application number: PCT/JP2021/012054
(87) International publication number: WO 2021/193659

(57) **Abstract**

The present invention provides a composition for treating or preventing renal cell cancer, the composition comprising, as an active ingredient, D-allose and/or a derivative thereof and/or a mixture thereof, and being characterized by being administered through the intestinal tract (for example, oral administration). Moreover, the present invention provides a method for treating or preventing renal cell cancer in a subject, the method comprising administering a composition, which is for treating or preventing renal cell cancer and contains D-allose as an active ingredient, to a subject in need thereof through the intestinal tract (for example, oral administration).

## Description

### FIELD

The invention relates to a composition for treatment or prevention of renal cell carcinoma which contains D-allose as an active ingredient and is administered through the intestinal tract, and to a method for treatment or prevention of renal cell carcinoma using it.

### BACKGROUND

At present, eradication of neoplastic cells in the body of patients undergoing treatment for cancer requires surgery, chemotherapy and/or radiation therapy. All of these approaches are generally onerous for patients. Known chemotherapy methods include antitumor drugs and anti-cancer agents, but their side-effects are generally problematic. It is therefore highly desirable to develop novel compounds, compositions and methods that are useful for treatment of cancer while either reducing or not producing such side-effects. There is also a need for treatment methods for cancer that provide therapy targeted more specifically to cancer cells and with lower toxicity.

There is currently increasing awareness and anticipation in regard to drugs and foods (functional foods) that not only treat but also prevent diseases. It is commonly known that certain "sugars" in foods are effective for treatment or prevention of disease, and in regard to the relationship between sugars and cancer, it has been reported that constipation can be eliminated and risk of colorectal cancer can be reduced by using the intestinal regulating effect of oligosaccharides, while it has also recently been reported that polysaccharides from *Agaricus* and the like have inhibiting effects on cancer. A relationship between sugar chains and cancer metastasis has been reported, but almost nothing has been published regarding whether "monosaccharides themselves" have an inhibiting effect on cancer cell proliferation. For example, PTL 1 describes "a colorectal cancer inhibitor having as the active ingredient watersoluble polysaccharides composed mainly of arabinoxylan" which is a polysaccharide effective for preventing cancer. It has also been reported that constipation can be eliminated and risk of colorectal cancer can be reduced by using the intestinal regulating effect of "oligosaccharides", while it has also recently been reported that polysaccharides from *Agaricus* and the like have inhibiting effects on cancer, and a relationship has also been reported between sugar chains and cancer metastasis.

One focus of attention in recent years has been on "rare sugars", as a subset of monosaccharides, whose physiological activity is gradually coming to light. Rare sugars are defined as "monosaccharides with low abundance in the natural world, and their derivatives", and expectations are high for their application and implementation in the field of medicine as well.

For example, PTL 2 reports on an *in vivo* antioxidant comprising the rare sugar D-allose, as an active ingredient. The pharmaceutical composition is used for mammals including humans, and comprises an *in vivo* antioxidant with D-allose as the active ingredient, while also being described as useful for treatment of liver cancer or skin cancer.

PTL 3 reports that in *in vitro* testing, D-allulose inhibits expression of the glucose transporter GLUT1 in cancer cells such as human liver cancer cells, human mammary gland cancer cells and human neuroblasts. GLUT1 is a glucose transporter expressed in normal cells as well, but its expression is known to be markedly increased in cancer cells, and therefore reducing expression of GLUT1 in cancer cells is expected to reduce uptake of D-glucose by cancer cells and to exhibit an anticancer effect.

However, while the rare sugar D-allulose has been reported to have an antitumor effect *in vitro* against human cancer cells, it has not yet been satisfactorily demonstrated that it can exhibit an anticancer effect of a useful level *in vivo,* and especially in the clinic.

### [CITATION LIST]

### [PATENT LITERATURE]

[PTL 1] Japanese Patent Publication No. 2787252
[PTL 2] Japanese Patent Publication No. 5330976
[PTL 3] International Patent Publication No. WO2016/152293

### SUMMARY

### [TECHNICAL PROBLEM]

It is an object of the present invention to provide a composition that is suitable for administration methods that are convenient with minimal distress, and that exhibits an effective anticancer effect.

### [SOLUTION TO PROBLEM]

As a result of substantial research, the present inventors have completed this invention upon being the first to find that, even by transintestinal administration such as oral administration, D-allose is efficiently taken up *in vivo* into engrafted renal cell carcinoma tissue and can exhibit an anticancer effect. Specifically, the present invention encompasses the following inventions.
[1] A composition for treatment or prevention of renal cell carcinoma which contains D-allose as an active ingredient and is administered through the intestinal tract.
[2] The composition according to claim 1, wherein the transintestinal administration is oral administration.
[3] The composition according to claim 1 or 2, wherein the D-allose is administered at 1 mg/kg body weight/day to 1000 mg/kg body weight/day.
[4] The composition according to any one of claims 1 to 3, wherein the D-allose is D-allose and/or a derivative thereof, and/or a mixture of the same.
[5] The composition according to claim 4, wherein the D-allose derivative is one or more D-allose derivatives selected from the group consisting of sugar alcohols in which the D-allose carbonyl group is an alcohol group, uronic acids in which the alcohol group of the D-allose is oxidized, amino sugars in which the alcohol group of the D-allose is replaced with an amino group, and D-allose derivatives in which any hydroxyl group of the D-allose is replaced with a hydrogen atom, halogen atom or an amino, carboxyl, nitro, cyano, lower alkyl, lower alkoxy, lower acyl, lower alkanoyloxy, lower alkoxycarbonyl, mono- or di- lower alkyl substituted amino, aralkyl, aryl or heteroaryl group.
[6] The composition according to any one of claims 1 to 5, which is a drug.
[7] The composition according to any one of claims 1 to 5, which is a food.
[8] The composition according to claim 7, wherein the food is a health functional food or dietary supplement.
[9] The composition according to claim 8, wherein the health functional food is a specified health food or nutritional function food.
[10] A method for treatment or prevention of renal cell carcinoma, comprising transintestinal administration of a composition for treatment or prevention of renal cell carcinoma containing D-allose as an active ingredient, to a subject in need thereof.
[11] The method according to claim 10, wherein the transintestinal administration is oral administration.
[12] The method according to claim 10 or 11, wherein the D-allose is administered at 1 mg/kg body weight/day to 1000 mg/kg body weight/day.
[13] The method according to any one of claims 10 to 12, wherein the D-allose is D-allose and/or a derivative thereof, and/or a mixture of the same.
[14] The method according to claim 13, wherein the D-allose derivative is one or more D-allose derivatives selected from the group consisting of sugar alcohols in which the D-allose carbonyl group is an alcohol group, uronic acids in which the alcohol group of the D-allose is oxidized, amino sugars in which the alcohol group of the D-allose is replaced with an amino group, and D-allose derivatives in which any hydroxyl group of the D-allose is replaced with a hydrogen atom, halogen atom or an amino, carboxyl, nitro, cyano, lower alkyl, lower alkoxy, lower alkanoyl, lower alkanoyloxy, lower alkoxycarbonyl, mono- or di- lower alkyl substituted amino, aralkyl, aryl or heteroaryl group.
[15] The method according to any one of claims 10 to 14, wherein the composition is orally administered as a drug.
[16] The method according to any one of claims 10 to 14, wherein the composition is orally administered as a food.
[17] The method according to claim 16, wherein the food is a health functional food or dietary supplement.
[18] The method according to claim 17, wherein the health functional food is a specified health food or nutritional function food.

### [ADVANTAGEOUS EFFECTS OF INVENTION]

As a result of much avid research, the present inventors have completed this invention upon finding that, even by transintestinal administration such as oral administration, D-allose is efficiently taken up *in vivo* into renal cell carcinoma cells and can exhibit an anticancer effect. This differs from methods of administration that must be conducted under the guidance of a doctor and that increase patient distress, such as intraperitoneal administration or intravenous injection, and therefore the present invention is highly superior from the viewpoint of safety, convenience and distress reduction. The invention also provides a novel treatment for renal cell carcinoma that is convenient and does not cause distress.

### BRIEF DESCRIPTION OF DRAWINGS

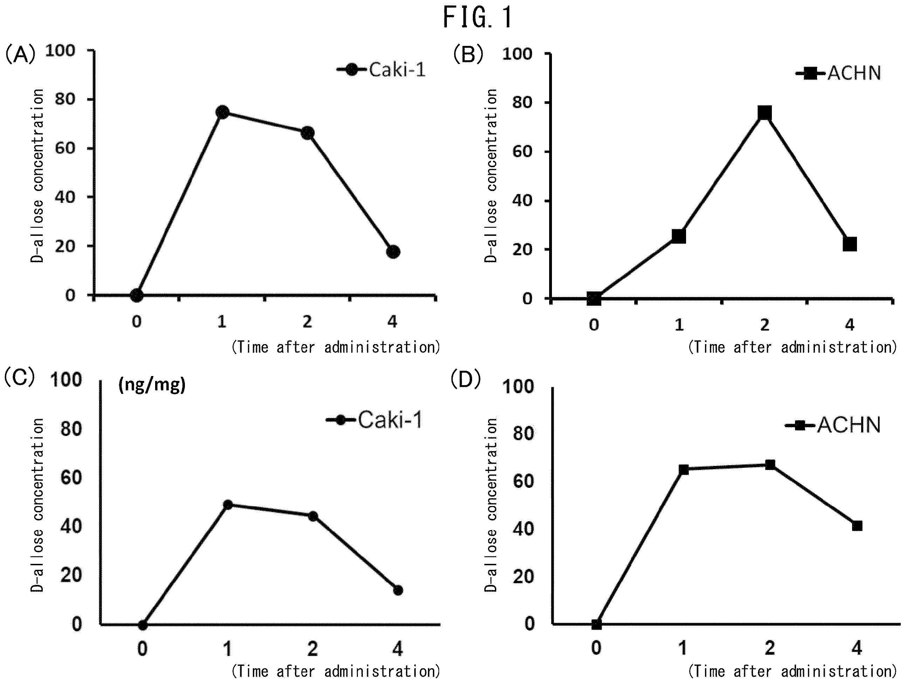

Fig. 1 is a graph showing change in tumor D-allose concentration after administration of D-allose to a renal cell carcinoma xenotransplantation mouse model. (A) shows tumor D-allose concentration after intraperitoneal administration (400 mg/kg body weight) of D-allose to a human renal cell carcinoma cell line (Caki-1) xenotransplantation mouse model, (B) shows tumor D-allose concentration after intraperitoneal administration (400 mg/kg body weight) of D-allose to a human renal cell carcinoma cell line (ACHN) xenotransplantation mouse model, (C) shows tumor D-allose concentration after oral administration (400 mg/kg body weight) of D-allose to a human renal cell carcinoma cell line (Caki-1) xenotransplantation mouse model, and (D) shows tumor D-allose concentration after oral administration (400 mg/kg body weight) of D-allose to a human renal cell carcinoma cell line (ACHN) xenotransplantation mouse model.
Fig. 2 is a graph showing change in tumor volume and change in mouse body weight in a renal cell carcinoma xenotransplantation mouse model by oral administration of D-allose. (A) shows change (%) in tumor volume, and (B) shows change (%) in mouse body weight. ∗P < 0.05.
Fig. 3 shows HE stained images (200x magnification) of tumor tissue extracted from a renal cell carcinoma xenotransplantation mouse model, for a control group and a D-allose-administered group. (A): control group, (B): 400 mg/kg body weight/day D-allose oral administration group.
Fig. 4 shows the results of comparing the degree of nuclear fission, calculated from HE stained images of tumor tissue extracted from a renal cell carcinoma xenotransplantation mouse model, for a control group and a D-allose-administered group.
Fig. 5 shows HE stained images (100x magnification) of kidney tissue extracted from a renal cell carcinoma xenotransplantation mouse model, for a control group and D-allose-administered group. (A): control group, (B): 400 mg/kg body weight/day D-allose oral administration group.
Fig. 6 shows HE stained images (100x magnification) of liver tissue extracted from a renal cell carcinoma xenotransplantation mouse model, for a control group and D-allose-administered group. (A): control group, (B): 400 mg/kg body weight/day D-allose oral administration group.
Fig. 7 is a graph showing change in tumor volume in a colorectal cancer xenotransplantation mouse model, by administration of D-allose. (A) Change in tumor volume (mm³) in colorectal cancer xenotransplantation mouse model by intraperitoneal administration of D-allose, (B) change in tumor volume (mm³) in colorectal cancer xenotransplantation mouse model by oral administration of D-allose. ∗P < 0.05. ∗∗∗P < 0.01.
Fig. 8 shows the results of comparing intracellular reactive oxygen species (ROS) production in a human renal cell carcinoma cell line (Caki-1, ACHN) with or without addition of D-allose. (A): Human renal cell carcinoma cell line Caki-1, (B): human renal cell carcinoma cell line ACHN.
Fig. 9 shows change in TXNIP expression level in a human renal cell carcinoma cell line (Caki-1, Caki-2) due to D-allose. (A): Human renal cell carcinoma cell line Caki-1, (B): human renal cell carcinoma cell line Caki-2.

### DESCRIPTION OF EMBODIMENTS

Embodiments for carrying out the invention will be described in detail below, with the understanding that the technical scope of the invention is not limited only to these embodiments. The prior art documents cited herein are incorporated in their entirety by reference throughout the present specification.

According to one aspect, the invention provides a composition for treatment or prevention of renal cell carcinoma which contains D-allose as an active ingredient and is administered through the intestinal tract. The composition of the invention may be provided as a drug or it may be provided as a food. The composition of the invention used as a food may be a health functional food (such as a specified health food or nutritional function food), or a dietary supplement, for example.

According to another aspect, the invention provides a method for treatment or prevention of renal cell carcinoma, which includes transintestinal administration of a composition for treatment or prevention of renal cell carcinoma containing D-allose as an active ingredient, to a subject in need thereof.

The D-allose to be used for the invention is in extremely low abundance in the natural world, compared to D-glucose (glucose) which is abundantly present. Of the monosaccharides that are the base units of sugars (a total of 34 monosaccharides with 6 carbon atoms (hexoses) exist, 16 of which are aldoses, 8 of which are ketoses and 10 of which are sugar alcohols), monosaccharides that are only present in trace amounts in the natural world (aldoses and ketoses) and their derivatives (sugar alcohols) are defined as "rare sugars", in contrast to "naturally-occurring monosaccharides", typically D-glucose (glucose), that are highly abundant in the natural world. The rare sugars that can be mass produced as of the current writing are D-psicose and D-allose. D-allose is the D-form of allose, classified as an aldose, and it is a hexose.

Previously disclosed methods for obtaining "D-allose" include a method of synthesis from D-psicose using L-rhamnose isomerase, and a method of reacting D-xylose isomerase with a D-psicose-containing solution, but the D-allose of the invention is not limited to these methods and may be obtained by any method of isomerization by chemical treatment. The D-psicose used as starting material for D-allose will usually be obtained by treating fructose with an enzyme (epimerase), but this is not limitative, and it can be obtained by a method using microorganisms that produce the enzyme, or it may be an extract from a natural product, or a substance that is present in a natural product, or a substance isomerized by a chemical treatment method. The method for purifying the D-psicose using an enzyme may be a publicly known method.

The D-allose may also be in the form of D-allose-containing syrup. A D-allose-containing syrup can be obtained by appropriately mixing common syrups (liquid sugar), but it is also sold in stores as a "food" such as the commercial product "Rare Sugar Sweet" (manufacturer: RareSweet Co., Ltd., vendor: Matsutani Chemical Industry Co., Ltd.), and is readily available.

The method of obtaining the D-allose-containing syrup may be, for example, reacting an alkali with a monosaccharide (D-glucose or D-fructose) to cause Lobry de Bruyn-Van Ekenstein transformation or retro-aldol reaction and subsequent aldol reaction (such reactions known as "alkali isomerization reaction"), the syrup containing the produced monosaccharides (including rare sugars) being generally referred to as "rare sugar-containing syrup", and D-glucose and/or D-fructose may be used as the starting material to obtain an alkali-isomerized syrup with a D-glucose and/or D-fructose content of 55 to 99 mass%. The product "Rare Sugar Sweet" is a syrup containing rare sugars obtained by the method disclosed in International Patent Publication No. WO2010/113785 with isomerized sugar as the starting material, and it was produced so as to contain primarily D-psicose and D-allose as the rare sugars. The rare sugars in the rare sugar-containing syrup obtained by this method are 0.5 to 17 mass% D-psicose and 0.2 to 10 mass% D-allose, as proportions with respect to the total sugars. A publication by Takahashi et al. (Ouyou Toushitsu Kagaku, Vol. 5, No. 1, 44-49(2015)) reports that it is a syrup containing 5.4 g/100 g D-psicose, 5.3 g/100 g D-sorbose, 2.0 g/100 g D-tagatose, 1.4 g/100 g D-allose and 4.3 g/100 g D-mannose.

The starting materials used to produce the rare sugar-containing syrup are starch, sugar, isomerized sugar, fructose and glucose. Isomerized sugar is widely considered to be a mixture of sugars with a main composition of D-glucose and D-fructose in a specified compositional ratio, and generally refers to syrup consisting mainly of glucose and fructose, obtained by glucose isomerase or alkali isomerization of a sugar solution composed mainly of glucose from hydrolysis of starch using an enzyme such as amylase or an acid. According to the JAS standard, the term "glucose-fructose syrup" is applied to a fructose content (percentage of fructose of the total sugars) of less than 50%, "fructose-glucose syrup" is applied for ≥50% and <90%, "high fructose syrup" is applied for ≥90%, and "sugar-containing fructose-glucose syrup" is applied for syrups with addition of sugar to glucose-fructose syrup in an amount not exceeding glucose-fructose syrup, and the starting material for the rare sugar-containing syrup of the invention may employ any of these isomerized sugars.

For example, rare sugar-containing syrup obtained using D-fructose as the starting material contains 5.2% D-psicose, 1.8% D-allose, 15.0% glucose and 69.3% D-fructose. Rare sugar-containing syrup obtained using isomerized sugar as the starting material contains 3.7% D-psicose, 1.5% D-allose, 45.9% glucose and 37.7% D-fructose, or when D-glucose is used as the starting material it contains 5.7% D-psicose, 2.7% D-allose, 47.4% glucose and 32.1% D-fructose, although the sugar composition will differ depending on differences in the starting material and treatment method. D-allose separated and purified from such syrups may also be used, or the syrups may be used directly.

According to one aspect, the D-allose to be used for the invention may be D-allose and/or its derivative, and/or a mixture thereof. Compounds whose molecular structure is altered from starting compounds by chemical reaction are generally referred to as derivatives of the starting compounds. Throughout the present specification, "D-allose derivative" is used to mean a compound obtained by converting the molecular structure of the D-allose starting compound by chemical reaction; and also includes compounds obtained by converting the molecular structure of an analog compound of D-allose (such as D-glucose) as the starting compound by chemical reaction, being a compound having the same structure as a compound obtained by converting the molecular structure of D-allose by chemical reaction (also referred to as "D-allose structural analog"). Derivatives of hexoses including D-allose are commonly known, such as sugar alcohols (reduction of monosaccharides converts aldehyde and ketone groups to alcohol groups, producing a polyhydric alcohol with the same number of carbon atoms), uronic acids (monosaccharides with an oxidized alcohol group, of which known natural examples include D-glucuronic acid, galacturonic acid and mannuronic acid), and amino sugars (having an OH group of the sugar molecule replaced with an NH₂ group, e.g., glucosamine, chondrosamine or a glycoside), though with no limitation to these. The D-allose derivative may also be a D-allose derivative selected from among sugar alcohols with the carbonyl group of D-allose converted to an alcohol group, uronic acids with an alcohol group of D-allose oxidized, and amino sugars with an alcohol group of D-allose replaced with an amino group.

According to another aspect, the D-allose derivative may be a D-allose derivative in which any hydroxyl group of D-allose (such as the hydroxyl group at position 2, position 3, position 4, position 5 and/or position 6) is replaced with a hydrogen atom, a halogen atom, an amino, carboxyl, nitro, cyano, lower alkyl, lower alkoxy, lower alkanoyl, lower alkanoyloxy or lower alkoxycarbonyl group, a mono- or di- lower alkyl substituted amino group, or an aralkyl, aryl or heteroaryl group.

Halogen atoms are the atoms fluorine, chlorine, bromine and iodine. The alkyl portion of a lower alkyl, lower alkoxy, lower alkoxycarbonyl or mono- or di- lower alkyl substituted amino group is a straight-chain, branched or cyclic C1-C6 alkyl group, with specific examples including methyl, ethyl, propyl, isopropyl, butyl, isobutyl, sec-butyl, tert-butyl, pentyl, isopentyl, neopentyl, hexyl, isohexyl, cyclopropyl, cyclobutyl, 2-methylcyclopropyl, cyclopropylmethyl, cyclopentyl and cyclohexyl.

The lower alkanoyl portion of a lower alkanoyl or lower alkanoyloxy group is a straight-chain, branched or cyclic C1-C7 alkanoyl group, with specific examples including formyl, acetyl, propionyl, butyryl, isobutyryl, valeryl, isovaleryl, pivaloyl, hexanoyl, cyclopropylcarbonyl, cyclobutylcarbonyl, 2-methylcyclopropylcarbonyl and cyclohexylcarbonyl.

An aralkyl group is a C7-C20 aralkyl group, with specific examples including benzyl, phenethyl, α-methylbenzyl, benzhydril, trityl and naphthylmethyl.

An aryl group is a C6-C14 aryl group, with specific examples including phenyl and naphthyl.

A heteroaryl group is a C3-C8 heteroaryl group, which is a monocyclic, polycyclic or fused ring of 1 to 4 N, O or S atoms which may be the same or different, with specific examples including 2-pyridyl, 3-pyridyl, 4-pyridyl, 2-quinonyl, 3-quinonyl, 4-quinonyl, 5-quinonyl, 6-quinonyl, 7-quinonyl, 8-quinonyl, 2-indolyl, 3-indolyl, 4-indolyl, 5-indolyl, 6-indolyl, 7-indolyl, 2-furyl, 3-furyl, 2-thienyl, 3-thienyl, 2-pyrrolidyl, 3-pyrrolidyl, 2-imidazolyl, 4-imidazolyl, 5-imidazolyl, 2-oxazolyl, 4-oxazolyl, 5-oxazolyl, 2-thiazolyl, 4-thiazolyl and 5-thiazolyl.

According to one aspect, the D-allose derivative to be used for the invention may be a D-allose derivative such as 2-deoxy-D-allose, 5-deoxy-D-allose, 6-deoxy-D-allose or 3-deoxy-D-allose (3-deoxy-D-glucose).

Through the present specification, "D-allose and/or a derivative thereof, and/or a mixture thereof' may also be shortened to simply "D-allose". The "D-allose and/or a derivative thereof, and/or a mixture thereof' to be used for the invention is also interpreted as including pharmacologically acceptable salts and/or hydrates.

Throughout the present specification, the term "food" means food in general, but in addition to common foods including health foods, it also includes health functional foods such as specified health foods and nutritional function foods, as well as dietary supplements (supplements and nutritional supplements), feeds and food additives. The composition for treatment or prevention of renal cell carcinoma according to the invention has a D-allose food as the active ingredient and is administered through the intestinal tract, and it may also be in the form of a sweetener, seasoning, food additive, food material, food or beverage, health food or beverage, or a drug, quasi drug or feed to be used for treatment or prevention of renal cell carcinoma, all of which forms may be used by transintestinal administration (such as oral administration) for treatment or prevention of renal cell carcinoma.

According to one aspect, the composition of the invention is administered through the intestinal tract. The term "transintestinal administration", as used herein, is a form of administration whereby the components in the composition of the invention are absorbed through the intestinal tract, and for example, oral administration or tube administration (such as intranasal (administered through a catheter inserted into the stomach, duodenum or jejunum); or through a fistula (administered to the stomach, duodenum or jejunum using a catheter inserted into a fistula created in the neck or abdominal region)), with oral administration being preferred for the invention. Since the composition of the invention can be orally administered, it allows treatment of renal cell carcinoma to be carried out conveniently and without distress.

According to one aspect, the invention allows D-allose to be administered at 1 mg/kg body weight/day to 1000 mg/kg body weight/day, with appropriate adjustment depending on age and symptoms. Since substances administered by transintestinal administration such as oral administration are usually delivered after having been absorbed through the gastrointestinal tract, the proportion delivered to the target tissue is usually much lower compared to direct injection (such as intraperitoneal injection or intravenous injection). However, the present inventors have found that even when administered at 1 mg/kg body weight/day to 1000 mg/kg body weight/day, the D-allose in the composition of the invention is taken up into renal cell carcinoma either at an equivalent rate or with approximately about 20% reduction compared to non-transintestinal administration such as intraperitoneal administration, allowing it to exhibit an anticancer effect. For colorectal cancer, oral administration of D-allose has not exhibited the anticancer effect observed with intraperitoneal administration. This suggests that transintestinally administered D-allose is efficiently delivered at least to renal cell carcinoma, allowing it to exhibit an anticancer effect. It can therefore be administered at a dosage of 1 mg/kg body weight/day to 1000 mg/kg body weight/day, such as 10 mg/kg body weight/day to 800 mg/kg body weight/day or 50 mg/kg body weight/day to 500 mg/kg body weight/day, as an amount that can be ingested for transintestinal administration.

When the rare sugar D-allose and/or a derivative thereof, and/or a mixture thereof is used as a component of a food, an effective dose of the rare sugar D-allose and/or a derivative thereof, and/or a mixture thereof, can be safely ingested in the course of routine dietary habit. The rare sugar D-allose is an aldose and is therefore a highly safe compound for administration to humans.

Throughout the present specification, the term "drug" is used to include drugs and quasi drugs.

The D-allose and/or a derivative thereof, and/or a mixture thereof in the composition of the invention may include suitable additives such as excipients, stabilizers, preservatives, binders or disintegrators, and may be provided by formulation, selecting a suitable dosage form such as tablets, powder, granules, capsules, a solution, syrup, elixir or an oily or aqueous suspension.

For a solid formulation, the D-allose and/or a derivative thereof, and/or a mixture thereof may include pharmaceutically acceptable additives, and for example, fillers or extenders, binders, disintegrators, dissolution accelerators, moistening agents or lubricants may be selected as necessary for mixing and formulation. An excipient, disintegrator, binder and lubricant may be added to and mixed with the D-allose of the invention and/or a derivative thereof, and/or a mixture thereof, and the mixture may then be compacted and molded. Lactose, starch and mannitol are commonly used as excipients. Calcium carbonate and carboxymethyl cellulose calcium are commonly used as disintegrators. Gum arabic, carboxymethyl cellulose and polyvinylpyrrolidone are used as binders. Talc and magnesium stearate are publicly known lubricants.

Tablets can be masked, or coated in a publicly known manner to make them enteric-coated formulations. Ethyl cellulose and polyoxyethylene glycol may be used as coating agents.

In addition to the aforementioned drugs (pharmaceutical compositions), the composition of the invention can also be provided as a food (such as a medical food, specified health food, health assisting food, health food, nutritional function food, supplement, dietary supplement or herbal tea). D-allose can be efficiently taken up into renal cell carcinoma by transintestinal administration at a dosage of 1 mg/kg body weight/day to 1000 mg/kg body weight/day (for example, 50 mg to 50 g/day for a 50 kg adult), without any particular effects on other tissues, thus allowing it to be used not only for treatment but also for prevention of renal cell carcinoma.

Renal cell carcinoma to which the invention may be applied includes not only primary renal cell carcinoma in the kidneys but also metastatic renal cell carcinoma. Subjects for which the invention may be used include ones with unresectable cancer such as metastatic advanced cancer or local advanced cancer. Subjects for which the invention may be used include animals, including humans (mammals such as humans, cows, pigs, dogs and cats, and birds such as chickens).

According to one aspect, the invention may be used together with a publicly known anticancer agent, radiation therapy and/or operation (such as surgery). Anticancer agents to be used with the invention are not particularly restricted, and examples include molecular targeted drugs, alkylating agents, antimetabolites, platinum formulations, hormone agents, topoisomerase inhibitors, microtubule anticancer drugs, immunostimulants and anti-cancer antibiotics, optionally in combinations. Molecular targeted drugs include low molecular compounds and antibodies, examples of which are immune checkpoint inhibitors (such as PD-1 inhibitor, PD-L1 inhibitor, CTLA-1 inhibitor, KIR inhibitor, LAG3 inhibitor, CD137 inhibitor and CCR4 inhibitor), EGFR inhibitors (such as anti-EGFR antibody), VEGFR inhibitors (such as anti-VEGFR antibody) and GD2 inhibitors (such as GD2 antibody). Examples of molecular targeted drugs include ibritumomab tiuxetan, nivolumab, ipirimab, pembrolizumab, durvalumab, avelumab, atezolizumab, tremelimumab, lirilumab, BMS986016, urelumab, imatinib, everolimus, erlotinib, gefitinib, sunitinib, cetuximab, sorafenib, dasatinib, tamibarotene, trastuzumab, trastuzumab emtansine, tretinoin, panitumumab, bevacizumab, bortezomib, lapatinib, rituximab, vemurafenib and alectinib. Examples of alkylating agents include ifosfamide, carboquone, cyclophosphamide, dacarbazine, thiotepa, temozolomide, nimustine, busulfan, procarbazine, melphalan and ranimustine. Examples of antimetabolites include enocitabine, capecitabine, carmofur, cladribine, gemcitabine, cytarabine, cytarabine ocfosfate, tegafur, tegafur-uracil, tegafur-gimeracil-oteracil potassium, doxifluridine, nelarabine, hydroxycarbamide, fluorouracil, fludarabine, pemetrexed, pentostatin, mercaptopurine and methotrexate. Examples of platinum formulations include oxaliplatin, carboplatin, cisplatin and nedaplatin. Examples of hormone agents include anastrozole, exemestane, estramustine, ethynylestradiol, chlormadinone, goserelin, tamoxifen, dexamethasone, toremifene, bicalutamide, flutamide, prednisolone, fosfestrol, mitotane, methyltestosterone, medroxyprogesterone, mepitiostane, leuprorelin and letrozole. Examples of topoisomerase inhibitors include irinotecan, etoposide and nogitecan. Examples of microtubule anticancer drugs include eribulin, docetaxel, nogitecan, paclitaxel, vinorelbine, vincristine, vindesine and vinblastine. Examples of immunostimulants include interferon-α, interferon-β, interferon-γ, interleukins, ubenimex, lentinan and dry BCG. Examples of anti-cancer antibiotics include actinomycin D, aclarubicin, amrubicin, idarubicin, epirubicin, zinostatin stimalamer, daunorubicin, doxorubicin, pirarubicin, bleomycin, peplomycin, mitomycin C, mitoxantrone and liposomal doxorubicin.

Radiation therapy to be used in combination with the cell composition for cancer treatment of the invention may be any radiation therapy method known to those skilled in the art.

### EXAMPLES

The present invention will now be explained in greater detail by examples, with the understanding that the invention is not limited in any way by the examples.

### [Example]

### <Method of preparing renal cell carcinoma xenotransplantation mice>

The experiment was conducted using two different human renal cell carcinoma cell lines (Caki-1, ACHN). Each of the cells was cultured in a moist environment at 37°C, 5% CO₂ using 10% fetal bovine serum and RPMI-1640 culture solution (2000 mg D-glucose/L) containing HEPES buffer and penicillin-streptomycin. The renal cell carcinoma xenotransplantation mouse model was prepared by adjusting cultured cells to a 1.0 × 10⁵ cell/mL cell suspension with MEM culture solution, injecting 0.1 mL into the femoral subcutaneous tissue of Female athymic nude mice (BALB/c nu/nu, 6 weeks old), and confirming a tumor volume of 200 mm³ or greater after 1 week, and it was used for the following experiment.

### <Change in tumor D-allose concentration after oral administration of D-allose to renal cell carcinoma xenotransplantation mouse model>

In a xenotransplantation mouse model of two different human renal cell carcinoma cell lines (Caki-1, ACHN), tumors were enucleated from the mice at the 1st hour, 2nd hour and 4th hour before and after administration of D-allose by intraperitoneal administration (Fig. 1(A) and (B)) or oral administration (Fig. 1(C) and (D)) of a solution of rare sugar D-allose prepared using physiological saline to 400 mg/kg/0.2 mL. The monosaccharides in the supernatant obtained by ultrasonic disruption of the tumors in 1 mL of PBS and centrifugal separation at 3000 rpm for 5 minutes were labeled with ABEE (4-aminobenzoic acid ethyl ester) and the D-allose was quantitatively analyzed by HPLC (high performance liquid chromatography) (Fig. 1).

Similar to the results with intraperitoneal administration of D-allose (Fig. 1(A) and (B)), D-allose was detected in all of the tumors from Caki-1 and ACHN cells by 1 hour after oral administration of D-allose to the renal cell carcinoma xenotransplantation mouse model (Fig. 1(C) and (D)). The maximum value was seen at the 1st hour after administration with Caki-1 and after the 2nd hour with ACHN, with the intratumoral concentration of D-allose falling afterwards (Fig. 1(C) and (D)). Surprisingly, with oral administration, D-allose was detected in the tumors at about 80% of the D-allose concentration with intraperitoneal administration. This suggests that even when administered orally, D-allose is efficiently delivered and taken up into renal cell carcinoma.

### <Change in tumor volume in renal cell carcinoma xenotransplantation mouse model by oral administration of D-allose>

A D-allose-containing solution was orally administered from day 1, where "day 0" was defined as the day when the tumor size of the renal cell carcinoma xenotransplantation mouse model prepared using Caki-1 as the human renal cell carcinoma cell line reached 200 mm³ or greater.

Either 0.2 mL physiological saline or 0.2 mL physiological saline dissolving 400 mg/kg D-allose was injected into the esophagus of mice divided into 2 groups (control group and D-allose group), through a Neraton catheter. The administration was carried out once a day for a total of 32 consecutive days. The mouse body weights and the long and short diameters of the tumors were measured twice a week. The tumor volumes were calculated as: tumor long diameter × short diameter × short diameter × 0.5. The livers and kidneys were extracted with the tumors from the mice on the 32nd day after the start of oral administration and used for the subsequent experiment.

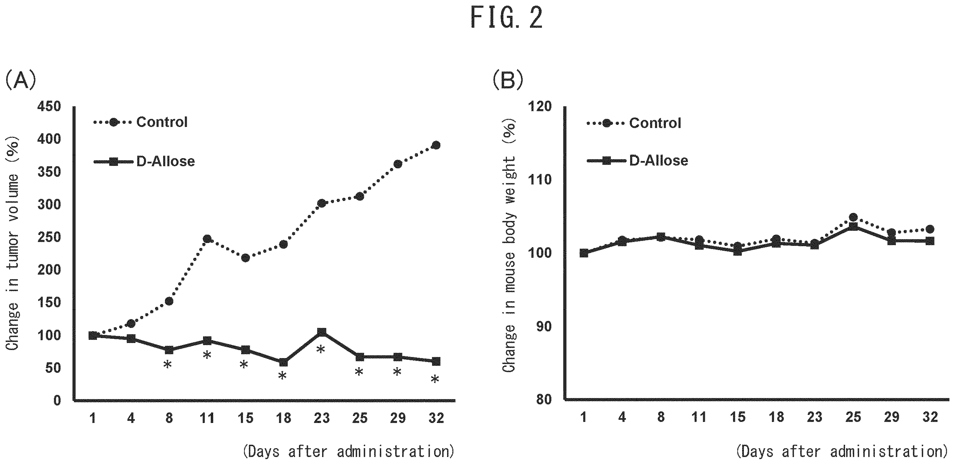

In the xenotransplantation mouse model prepared using Caki-1 cells, the tumor volume in the D-allose-administered group was significantly reduced after the 8th day compared to the control group (Fig. 2(A)). Tumor volume at the 32nd day in the D-allose-administered group was also significantly reduced compared to tumor volume before D-allose administration, and therefore the results showed promise of a tumor shrinkage effect in addition to a tumor growth inhibition effect by oral administration of D-allose (Mann-Whitney U test). There was no significant difference in body weight between the control group and D-allose-administered group during the observation period (Fig. 2(B)).

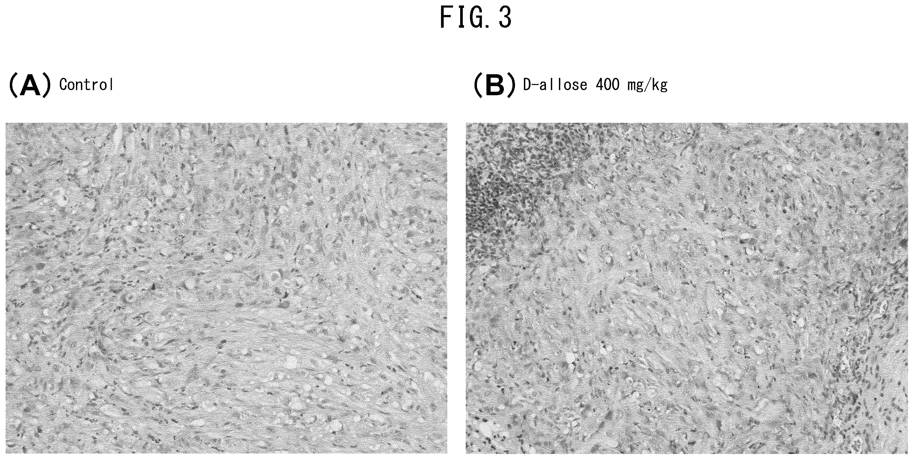

The tumors, kidneys and livers were extracted from the renal cell carcinoma xenotransplantation mouse models (control group and D-allose-administered group, 32nd day after start of administration), and the tissues were fixed with 4% paraformaldehyde/phosphate buffer, embedded in paraffin and sliced to thicknesses of 4 µm. The thin slices were stained with hematoxylin-eosin staining (HE staining) and observed (Fig. 3, Fig. 5 and Fig. 6).

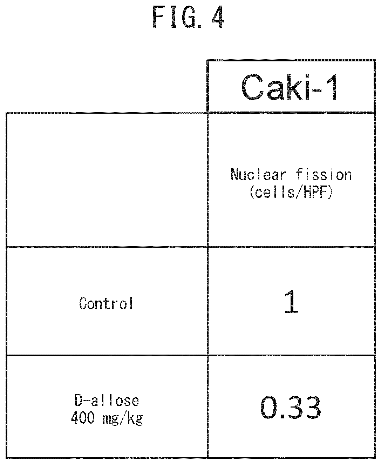

As a result of evaluation by a pathology specialist, the tumors extracted from the D-allose-administered group and renal cell carcinoma xenotransplantation mouse model (Caki-1 cells) were confirmed to have reduced nuclear fission (Fig. 3 and Fig. 4).

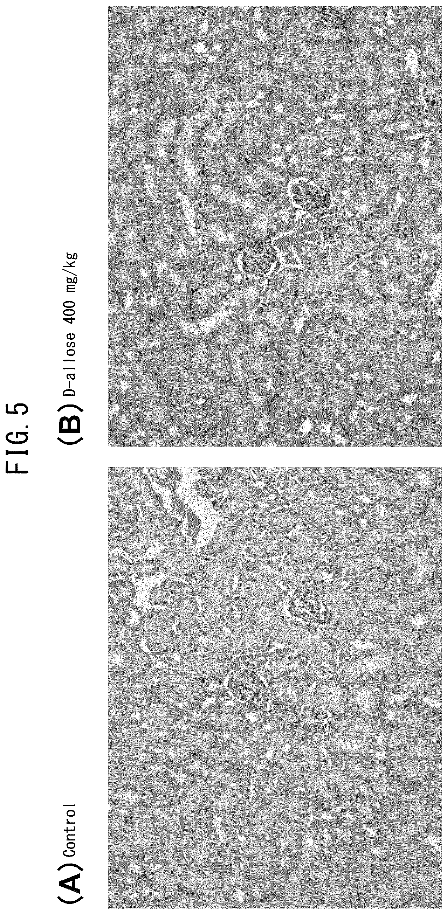

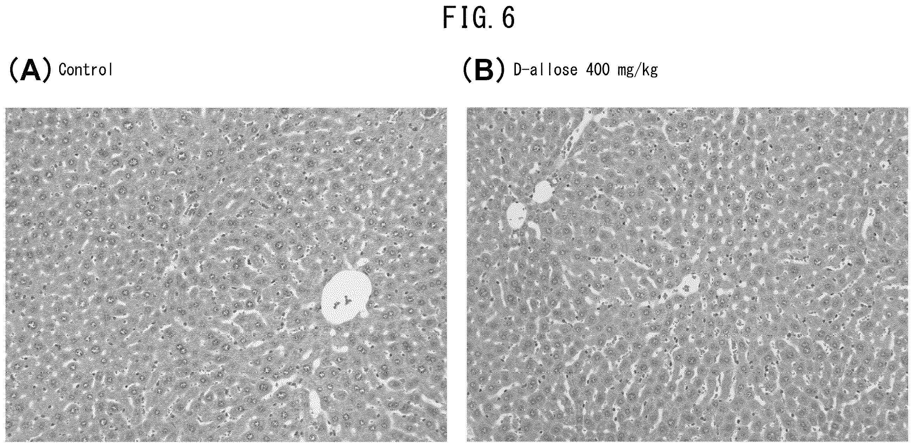

No particular difference was seen in the HE staining images of the kidney tissue and liver tissue extracted from the control group and D-allose-administered group (Fig. 5 and Fig. 6), suggesting that D-allose has no effect on kidney tissue or liver tissue.

### [Comparative Example]

### <Method of preparing colorectal cancer xenotransplantation mice>

A cell suspension containing 2.0 × 10⁶ cells of a human colorectal cancer cell line (DLD-1) was injected into femoral subcutaneous tissue of Male athymic nude mice (BALB/c Nude (nu/nu)mice), and adjustment of D-allose or D-glucose was initiated with "day 0" defined as the day when the tumor volume reached about 100 to 150 mm³.

### <Change in tumor volume in colorectal cancer xenotransplantation mouse model by intraperitoneal administration of D-allose>

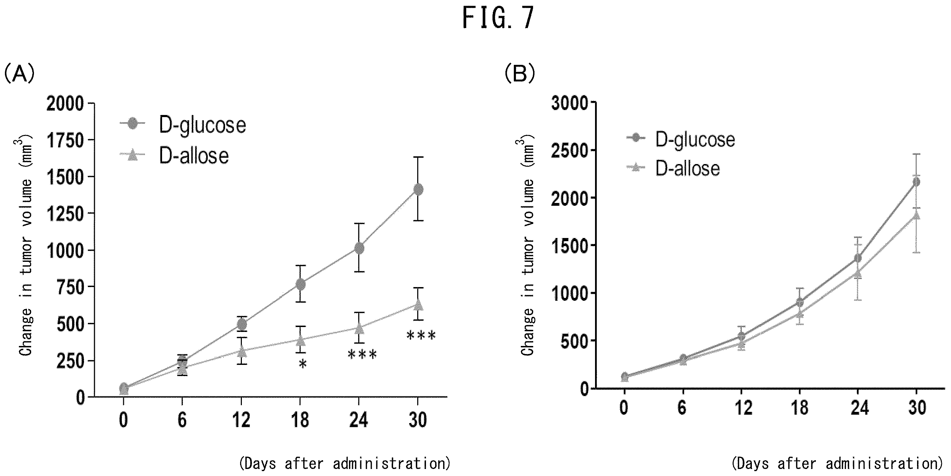

D-allose or D-glucose was intraperitoneally administered at 400 mg/kg to colorectal cancer xenotransplantation mice whose tumor volumes had reached about 100 mm³. Physiological saline was used as the solvent for adjustment to 0.2 ml, and administration was once per day for a total of 30 days. The tumor volumes were measured every 6 days. As a result, tumor volume in the D-allose-administered group was significantly smaller after the 18th day from the start of administration, compared to the D-glucose group (Mann-Whitney U test) (Fig. 7(A)).

### <Change in tumor volume in colorectal cancer xenotransplantation mouse model by oral administration of D-allose>

D-allose or D-glucose was orally administered at 100 mg/kg to colorectal cancer xenotransplantation mice whose tumor volumes had reached about 100 to 150 mm³. Distilled water was used as the solvent for adjustment to 0.2 ml, and administration was once per day for a total of 30 days. The tumor volumes were measured every 6 days. As a result, there was no significant difference in tumor volume between the D-allose-administered group and D-glucose group (Mann-Whitney U test) (Fig. 7(B)).

### <Effect of D-allose on intracellular reactive oxygen species (ROS) production in human renal cell carcinoma cell lines (Caki-1, ACHN)>

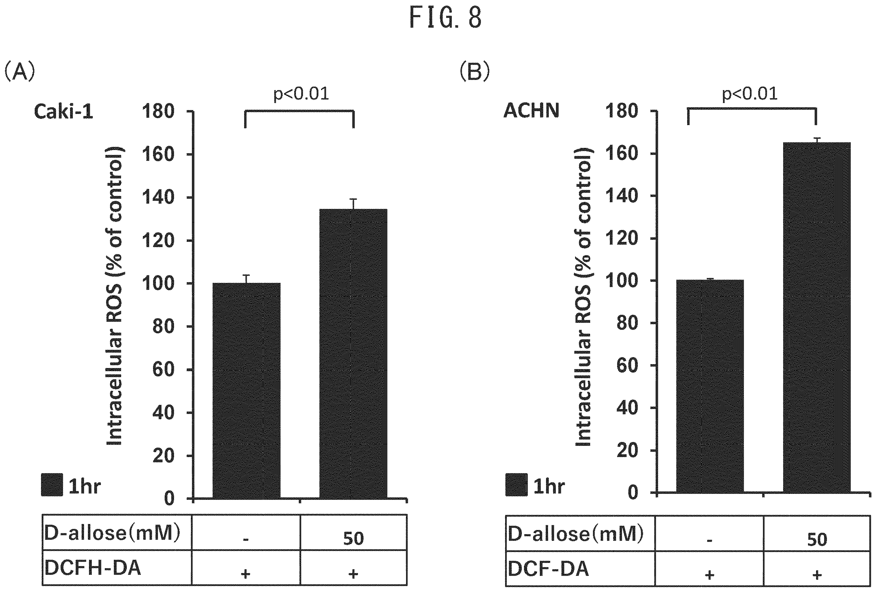

After seeding human renal cell carcinoma cell line (Caki-1 or ACHN) at 5.0 × 10⁵ in a 100 mm dish, culturing for 24 hours and then co-culturing with DMSO or D-allose (50 mM) for 1 hour, 10 µM 2,7-Dichlorofluorescin diacetate (DCF-DA; D6883, Sigma-Aldrich, USA) was added and the mixture was allowed to stand at 37°C for 30 minutes. The cells were recovered with trypsin treatment and collected by centrifugal separation for 5 minutes at 3500 × g, after which they were resuspended in ice-cold PBS and a CytoFLEX S (Beckman Coulter, CA, USA) was used to detect the DCF-DA fluorescence. Each experiment was conducted 3 times, and the data were analyzed using CytExpert software and recorded as the average value ±SE (Fig. 8).

The results clearly demonstrated that intracellular ROS was significantly increased by co-culturing with 50 mM D-allose for 1 hour in the two human renal cell carcinoma cell lines.

### <Effect of D-allose on TXNIP expression in human renal cell carcinoma cell lines (Caki-1, Caki-2)>

A human renal cell carcinoma cell line (Caki-1 or Caki-2) was seeded at 5.0 × 10⁵ in a 100 mm dish and cultured for 24 hours, after which it was co-cultured with D-allose (10, 25 or 50 mM). After 48 hours, the cells were recovered and the protein was extracted, and the concentration of each protein was measured using a Bio-Rad Protein Assay Kit (Bio-Rad, Laboratories, Inc. USA). A protein sample (40 µg) was electrophoresed on 10% Mini-PROTAN TGX Precast Gel (Bio-Rad) and a Trans-Blot Turbo transfer system (Bio-Rad) was used for transfer onto a PVDF Western Blotting Membrane. After blocking treatment for 1 hour (Superblock T20, Thermo, Rockford. IL, USA), anti-TXNIP antibody (D5F3E, CST, USA. 1: 1000) was used as primary antibody for reaction using an iBind Flex Western System (Thermo). The loading control used was β-actin (ab8227, abcam, CamBridge, UK, 1:1000).

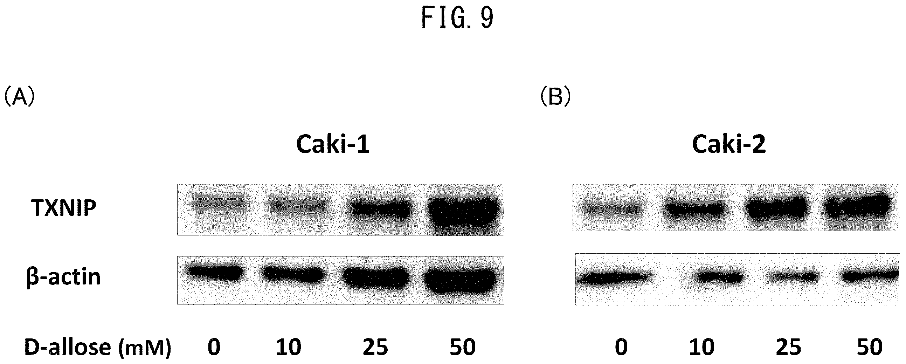

The results clearly demonstrated that D-allose increases TXNIP expression in a D-allose dose-dependent manner in the two human renal cell carcinoma cell lines (Fig. 9).

## Claims

1. A composition for treatment or prevention of renal cell carcinoma which contains D-allose as an active ingredient and is administered through the intestinal tract.

2. The composition according to claim 1, wherein the transintestinal administration is oral administration.

3. The composition according to claim 1 or 2, wherein the D-allose is administered at 1 mg/kg body weight/day to 1000 mg/kg body weight/day.

4. The composition according to any one of claims 1 to 3, wherein the D-allose is D-allose and/or a derivative thereof, and/or a mixture of the same.

5. The composition according to claim 4, wherein the D-allose derivative is one or more D-allose derivatives selected from the group consisting of sugar alcohols in which the D-allose carbonyl group is an alcohol group, uronic acids in which the alcohol group of the D-allose is oxidized, amino sugars in which the alcohol group of the D-allose is replaced with an amino group, and D-allose derivatives in which any hydroxyl group of the D-allose is replaced with a hydrogen atom, halogen atom or an amino, carboxyl, nitro, cyano, lower alkyl, lower alkoxy, lower alkanoyl, lower alkanoyloxy, lower alkoxycarbonyl, mono- or di- lower alkyl substituted amino, aralkyl, aryl or heteroaryl group.

6. The composition according to any one of claims 1 to 5, which is a drug.

7. The composition according to any one of claims 1 to 5, which is a food.

8. The composition according to claim 7, wherein the food is a health functional food or dietary supplement.

9. The composition according to claim 8, wherein the health functional food is a specified health food or nutritional function food.

10. A method for treatment or prevention of renal cell carcinoma, comprising transintestinal administration of a composition for treatment or prevention of renal cell carcinoma containing D-allose as an active ingredient, to a subject in need thereof.

11. The method according to claim 10, wherein the transintestinal administration is oral administration.

12. The method according to claim 10 or 11, wherein the D-allose is administered at 1 mg/kg body weight/day to 1000 mg/kg body weight/day.

13. The method according to any one of claims 10 to 12, wherein the D-allose is D-allose and/or a derivative thereof, and/or a mixture of the same.

14. The method according to claim 13, wherein the D-allose derivative is one or more D-allose derivatives selected from the group consisting of sugar alcohols in which the D-allose carbonyl group is an alcohol group, uronic acids in which the alcohol group of the D-allose is oxidized, amino sugars in which the alcohol group of the D-allose is replaced with an amino group, and D-allose derivatives in which any hydroxyl group of the D-allose is replaced with a hydrogen atom, halogen atom or an amino, carboxyl, nitro, cyano, lower alkyl, lower alkoxy, lower alkanoyl, lower alkanoyloxy, lower alkoxycarbonyl, mono- or di- lower alkyl substituted amino, aralkyl, aryl or heteroaryl group.

15. The method according to any one of claims 10 to 14, wherein the composition is orally administered as a drug.

16. The method according to any one of claims 10 to 14, wherein the composition is orally administered as a food.

17. The method according to claim 16, wherein the food is a health functional food or dietary supplement.

18. The method according to claim 17, wherein the health functional food is a specified health food or nutritional function food.
